# EUROPÄISCHE PATENTANMELDUNG

(11) **EP 1 790 298 A1**
(43) Veröffentlichungstag der Anmeldung: **30.05.2007**
(21) Anmeldenummer: 06123897.8
(22) Anmeldetag: 11.11.2006
(51) Int. Cl.: A61B 17/16, A61B 17/17

(54) **Chirurgisches Führungsinstrument**

(30) Priorität: 24.11.2005 DE 102005056824
(71) Anmelder: Aesculap AG & Co. KG, 78532 Tuttlingen (DE)
(72) Erfinder: Fischer, Kay, 78532, Tuttlingen (DE); Schultz, Robert, 78532, Tuttlingen (DE)
(74) Vertreter: Hoeger, Stellrecht & Partner Patentanwälte

(57) **Zusammenfassung**

Um ein chirurgisches Führungsinstrument für ein chirurgisches Bearbeitungswerkzeug, insbesondere einen Meißel, mit einem Implantatkörper zum Einführen in einen Zwischenwirbelraum zwischen benachbarte Wirbelkörper einer menschlichen oder tierischen Wirbelsäule, mit einem Schaft und mit einer Anschlagvorrichtung zum Begrenzen einer Einführtiefe des Implantatkörpers in den Zwischenwirbelraum, wobei der Schaft ein distales und ein proximales Ende aufweist, wobei der Implantatkörper zwei an den benachbarten Wirbelkörpern anlegbare Anlageflächen definiert und am distalen Ende des Schafts angeordnet ist, wobei die Anschlagvorrichtung mindestens einen am distalen Ende des Schafts oder am Implantatkörper beweglich gelagerten Anschlag aufweist, der eine quer oder im Wesentlichen quer zu mindestens einer der Anlageflächen in distaler Richtung weisende Anschlagfläche aufweist, so zu verbessern, dass eine Einführtiefe für ein chirurgisches Bearbeitungswerkzeug auf einfache Weise eingestellt werden kann, wird vorgeschlagen, dass die Anschlagvorrichtung ein am proximalen Ende oder im Bereich des proximalen Endes des Schafts angeordnetes Betätigungsglied und ein am Schaft gelagertes Kraftübertragungsglied umfasst und dass das Betätigungsglied, das Kraftübertragungsglied und der mindestens eine Anschlag derart angeordnet und zusammenwirkend ausgebildet sind, dass durch Betätigung des Betätigungsglieds der mindestens einen Anschlag in proximaler und/oder distaler Richtung bewegbar ist.

## Beschreibung

Die vorliegende Erfindung betrifft ein chirurgisches Führungsinstrument für ein chirurgisches Bearbeitungsinstrument, insbesondere einen Meißel mit einem Implantatkörper zum Einführen in einen Zwischenwirbelraum zwischen benachbarte Wirbelkörper einer menschlichen oder tierischen Wirbelsäule, mit einem Schaft und mit einer Anschlagvorrichtung zum Begrenzen einer Einführtiefe des Implantatkörpers in den Zwischenwirbelraum, wobei der Schaft ein distales und ein proximales Ende aufweist, wobei der Implantatkörper zwei an den benachbarten Wirbelkörpern anlegbare Anlageflächen definiert und am distalen Ende des Schafts angeordnet ist, wobei die Anschlagvorrichtung mindestens einen am distalen Ende des Schafts oder am Implantatkörper beweglich gelagerten Anschlag aufweist, der eine quer oder im Wesentlichen quer zu mindestens einer der Anlageflächen in distaler Richtung weisende Anschlagfläche aufweist.

Häufige Erkrankungen im Bereich der Wirbelsäule sind degenerative Veränderungen von Bandscheiben, die benachbarte Wirbelkörper der Wirbelsäule gelenkig miteinander verbinden. In Fällen, in denen diese so weit degeneriert sind, dass sie nicht mehr erhalten werden können, werden sie häufig durch künstliche Bandscheiben, so genannte Zwischenwirbelimplantate, ersetzt. Diese können benachbarte Wirbelkörper starr miteinander verbinden oder auch gelenkig, je nach Aufbau des Zwischenwirbelimplantats. Derartige Zwischenwirbelimplantate sind häufig mit von Anlageflächen, die zur Anlage an an den Zwischenwirbelraum angrenzende Wirbelkörperflächen ausgebildet sind, abstehenden finnenartigen Vorsprüngen versehen, die eine Relativbewegung zwischen dem Zwischenwirbelimplantat und den Wirbelkörpern, an denen sie anliegen, verhindern. Um beim Einbringen des einen oder zwei oder mehrere finnenartige Vorsprünge aufweisenden Zwischenwirbelimplantats in den Zwischenwirbelraum eine Beschädigung, beispielsweise durch Spalten des jeweiligen Wirbel körpers zu verhindern, werden vor dem Einsetzen des Zwischenwirbelimplantats an den Wirbelkörpern Nuten eingearbeitet, beispielsweise gefräst oder mit einem Meißel eingeschlagen, in die die finnenartigen Vorsprünge des Zwischenwirbelimplantats eintauchen können. Ein Problem dabei ist, dass das Bearbeitungswerkzeug, beispielsweise ein Meißel oder ein Fräswerkzeug, zu weit in den Zwischenwirbelraum von ventral her kommend in Richtung auf den Spinalkanal der Wirbelsäule vorgeschoben wird. Um dies zu vermeiden sind Instrumente bekannt, die einen Anschlag aufweisen, der verhindert, dass das Führungsinstrument zu weit in den Zwischenwirbelraum eingeführt werden kann, so dass eine Einführ- oder Einschlagtiefe eines Bearbeitungswerkzeugs begrenzt wird, und zwar durch die Anschlagvorrichtung des Instruments.

Die Anschlagvorrichtungen bekannter Instrumente sind direkt an einem distalen Ende des Instruments angeordnet und können nur vor dem Einführen des Implantatkörpers des Führungsinstruments in den Zwischenwirbelraum eingestellt werden. Zur Variation einer Einschlagtiefe oder Einführtiefe des chirurgischen Bearbeitungswerkzeugs muss das Führungsinstrument jedes Mal aus dem Körper des Patienten entfernt werden. Oder es muss ein so großer Zugang zum Körper geschaffen werden, der es gestattet, die Anschlagvorrichtung zu verstellen. Ein minimalinvasiver Eingriff wäre so praktisch unmöglich.

Es ist daher Aufgabe der vorliegenden Erfindung, ein chirurgisches Führungsinstrument der eingangs beschriebenen Art so zu verbessern, dass eine Einführtiefe für ein chirurgisches Bearbeitungswerkzeug auf einfache Weise eingestellt werden kann.

Diese Aufgabe wird bei einem chirurgischen Führungsinstrument der eingangs beschriebenen Art erfindungsgemäß dadurch gelöst, dass die Anschlagvorrichtung ein am proximalen Ende oder im Bereich des proximalen Endes des Schafts angeordnetes Betätigungsglied und ein am Schaft gelagertes Kraftübertragungsglied umfasst und dass das Betätigungsglied, das Kraftübertragungsglied und der mindestens eine Anschlag derart angeordnet und zusammenwirkend ausgebildet sind, dass durch Betätigung des Betätigungsglieds der mindestens eine Anschlag in poximaler und/oder distaler Richtung bewegbar ist.

Die erfindungsgemäße Weiterbildung hat den Vorteil, dass die Präparierung der Wirbelkörper vor dem Einsetzen eines Zwischenwirbelimplantats mit finnenartigen Vorsprüngen wesentlich vereinfacht wird. Insbesondere kann das Führungsinstrument eingesetzt werden, und zwar durch einen minimalinvasiven Zugang, und nach dem Einsetzen kann die Anschlagvorrichtung durch das Betätigungsglied von außerhalb des menschlichen oder tierischen Körpers betätigt werden, um den Anschlag zu verstellen. Durch Verändern einer Relativposition zwischen Anschlag und Implantatkörper des Führungsinstruments kann somit auch eine maximale Einführtiefe eines chirurgischen Bearbeitungswerkzeugs beliebig verändert werden. Beispielsweise kann dies unter Röntgenkontrolle geschehen, so dass sichergestellt werden kann, dass beim Bearbeiten der an den Zwischenwirbelraum angrenzenden Wirbelkörper nur so viel vom jeweiligen Wirbelkörper abgetragen wird, wie tatsächlich benötigt wird, um das Zwischenwirbelimplantat einzusetzen. Des Weiteren wird so vermieden, dass beim Präparieren der Wirbelkörper das Bearbeitungswerkzeug an oder in den Spinalkanal gelangen und zu Verletzungen führen kann.

Vorteilhafterweise ist das Betätigungsglied mit dem Kraftübertragungsglied lösbar verbindbar. Dies gestattet es, das Führungsinstrument auch zu Reinigungszwecken zu zerlegen. Ferner kann so auch das Betätigungsglied abgenommen und beispielsweise durch ein anderes Instrument ersetzt werden.

Ferner ist es auch denkbar, ein Betätigungsglied vorzusehen, mit dem mehrere verschiedene Führungsinstrumente verbunden werden können.

Günstig ist es, wenn eine Kupplungsvorrichtung vorgesehen ist zum Verbinden des Kraftübertragungsglieds mit dem Betätigungsglied, wenn ein erstes und ein mit diesem in einer Kupplungsstellung in Eingriff bringbares zweites Kupplungsglied vorgesehen sind, wenn das erste Kupplungsglied am Betätigungsglied angeordnet ist und wenn das zweite Kupplungsglied am Kraftübertragungsglied angeordnet ist. Durch diese Weiterbildung lässt sich das Betätigungsglied auf einfache Weise mit dem Kraftübertragungsglied verbinden.

Dabei kann es vorteilhaft sein, wenn das eine der beiden Kupplungsglieder ein Außenvielkant oder ein Außenvielrund ist und wenn das andere der beiden Kupplungsglieder ein korrespondierender Innenvielkant oder ein Innenvielrund ist. Dies ermöglicht eine form- und/oder kraftschlüssige Verbindung zwischen dem Betätigungsglied und dem Kraftübertragungsglied, so dass infolge einer Bewegung des Betätigungsglieds eine entsprechende Bewegung des Kraftübertragungsglieds sicher erreicht werden kann. Beispielsweise kann das Kraftübertragungsglied verschiebbar und/oder verdrehbar am Schaft gelagert sein.

Um das Führungsinstrument auf einfache Weise handhaben zu können, ist es günstig, wenn am proximalen Ende des Schafts ein Griffteil vorgesehen ist.

Vorteilhafterweise ist das Griffteil mit dem Schaft lösbar verbindbar. Dies hat insbesondere den Vorteil, dass das Griffteil, wenn es nicht benötigt wird, beispielsweise dann, wenn der Implantatkörper in gewünschter Weise in den Zwischenwirbelraum eingeführt ist, entfernt werden kann, um im Operationsfeld nicht zu stören. Ferner hat dies auch den Vorteil, dass ein Griffteil für unterschiedliche Schäfte vorgesehen sein kann. Dies bedeutet, dass beispielsweise in einem Operationssaal nur ein einziges Griffteil erforderlich ist und dass ein Satz unterschiedlicher Schäfte mit unterschiedlichen Implantatkörpern vorgesehen sein kann, so dass individuell unterschiedliche Führungsinstrumente in Abhängigkeit der Größe des einzusetzenden Zwischenwirbelimplantats ausgewählt werden können.

Besonders einfach lässt sich der Schaft mit dem Griffteil verbinden, wenn eine Verbindungsvorrichtung vorgesehen ist zum Verbinden des Schafts mit dem Griffteil, wenn ein erstes und ein mit diesem in einer Verbindungsstellung in Eingriff bringbares zweites Verbindungsglied vorgesehen sind, wenn das erste Verbindungsglied am Schaft angeordnet ist und wenn das zweite Verbindungsglied am Griffteil angeordnet ist.

Auf einfache Weise lässt sich eine formschlüssige und/oder kraftschlüssige Verbindung zwischen dem Schaft und dem Griffteil erreichen, wenn das eine der beiden Verbindungsglieder ein Außenvielkant oder ein Außenvielrund ist und wenn das andere der beiden Verbindungsglieder ein korrespondierender Innenvielkant oder ein Innenvielrund ist.

Insbesondere kann es günstig sein, wenn der Außenvielkant einen kurzen Schaftabschnitt des Schafts bildet und wenn der Innenvielkant am Griffteil angeordnet ist. Dies ermöglicht es, dass der Schaft teilweise, beispielsweise mit seinem proximalen Ende, in das Griffteil eingeführt wird. Der Innenvielkant bildet daher eine Schaftaufnahme für den Außenvielkant aus.

Um das Griffteil und den Schaft auf einfache Weise voneinander trennen zu können, ist es günstig, wenn die Verbindungsvorrichtung in Form einer Rastverbindung ausgebildet ist, wenn die beiden Verbindungsglieder in Form von in einer Raststellung in Eingriff stehenden Rastgliedern ausgebildet sind und wenn die beiden Rastglieder derart angeordnet sind, dass sie relativ zueinander bewegbar sind beim Übergang von der Raststellung in eine Lösestellung, in der sie außer Eingriff stehen, und umgekehrt. Beispielsweise können mit einer solchen Rastverbindung der Schaft und das Griffteil auf einfache Weise zusammengesteckt werden. Zum Lösen der Rastverbindung müssen nur die beiden in der Raststellung in Eingriff stehenden Rastglieder relativ zueinander so bewegt werden, dass sie außer Eingriff gelangen. Dann können der Schaft und das Griffteil relativ zueinander bewegt und voneinander getrennt werden.

Vorteilhaft ist es, wenn eines der Rastglieder in Form einer am Schaft angeordneten Rastausnehmung ausgebildet ist und wenn das andere Rastglied eine federnd vorgespannte, am Griffteil gelagerte Rastklinke ist. Selbstverständlich können die beiden Rastglieder auch umgekehrt am Schaft oder am Griffteil angeordnet sein. Die vorgeschlagene Ausbildung hat jedoch den Vorteil, dass der Schaft besonders einfach ausgebildet und besonders einfach herzustellen ist, so dass insbesondere auch nur ein Griffteil für unterschiedliche Schäfte bereitgestellt werden muss, wodurch sich Kosten bei der Herstellung eines Satzes unterschiedlicher Führungsinstrumente deutlich reduzieren lassen.

Um sicher zu verhindern, dass sich der Schaft unbeabsichtigt vom Griffteil löst, ist es vorteilhaft, wenn die Rastklinke in einer Richtung quer zur Längsachse des Schafts bewegbar gelagert ist. So können auf das Führungsinstrument wirkende Kräfte in Richtung der Längsachse des Schafts nicht dazu führen, dass sich das Griffteil vom Schaft lösen kann.

Grundsätzlich wäre es denkbar, das Betätigungsglied am Schaft anzuordnen. Bei einem Satz unterschiedlicher Führungsinstrumente müsste dann an jedem Schaft auch ein Betätigungsglied vorgesehen sein. Daher ist es vorteilhaft, wenn das Betätigungsglied am Griffteil angeordnet ist. Dadurch kann darauf verzichtet werden, an jedem Schaft ein Betätigungsglied vorzusehen. Ist das Griffteil vom Schaft lösbar, so kann damit auch das Betätigungsglied vom Schaft und vom Kraftübertragungsglied gelöst werden.

Denkbar wäre es, dass das Kraftübertragungsglied in Richtung der Längsachse des Schafts verschiebbar an diesem gelagert ist. Günstig ist es, wenn das Kraftübertragungsglied eine am Schaft rotierbar gelagerte Antriebswelle ist. Dadurch wird eine Bewegung des Kraftübertragungsglieds und des Schafts relativ zueinander in Richtung der Längsachse verhindert. Insbesondere eignet sich die rotierbar gelagerte Antriebswelle, um vom Betätigungsglied in Rotation versetzt zu werden und um den mindestens einen Anschlag direkt oder indirekt zu bewegen.

Denkbar wäre es, das Betätigungsglied als parallel zur Längsachse des Schafts verschiebbares Schub- und Zugglied vorzusehen. Besonders günstig ist es jedoch, wenn das Betätigungsglied ein um eine Längsachse des Schafts rotierbar gelagertes Antriebsrad ist. Dadurch kann es auf einfache Weise mit einem am Schaft rotierbar gelagerten Kraftübertragungsglied verbunden werden. Zudem lässt es sich besonders einfach und ergonomisch betätigen, da für eine Rotation des Betätigungsglieds keine Kräfte in Richtung der Längsachse auf das Führungsinstrument einwirken, die zu einer Positionsänderung desselben führen könnten.

Vorzugsweise ist am Implantatkörper eine Meißelführung vorgesehen. Diese kann zum Einführen eines Meißels oder eines anderen Bearbeitungswerkzeugs dienen, mit dem ein Wirbelkörper bearbeitet, insbesondere teilweise reseziert werden kann.

Ein besonders einfacher Aufbau des Führungsinstruments ergibt sich, wenn die Meißelführung mindestens eine Führungsnut umfasst und wenn die mindestens eine Führungsnut in einer der beiden Anlageflächen parallel oder im Wesentlichen parallel zur Längsachse verlaufend angeordnet ist. Ein Bearbeitungswerkzeug, beispielsweise ein Meißel, kann so auf einfache Weise in der Führungsnut geführt werden, um einen Teil eines Wirbelkörpers zu resezieren, um eine Vertiefung zur Aufnahme eines finnenartigen Vorsprungs an einem Zwischenwirbelimplantat zu bilden.

Eine optimale Führung auf einer möglichst langen Strecke lässt sich erreichen, wenn mindestens ein Teil der Meißelführung proximalseitig vom Implantatkörper vorsteht. Der proximal vorstehende Teil kann gleichzeitig als Anschlag für das Bearbeitungswerkzeug dienen, wenn es in der Meißelführung relativ zum Implantatkörper bewegt wird.

Damit insbesondere ein Doppelmeißel mit zwei Klingen als Bearbeitungswerkzeug verwendet werden kann, um gleichzeitig an beiden benachbart zum Zwischenwirbelraum angeordneten Wirbelkörpern jeweils eine Aufnahme für einen Führungsvorsprung am Zwischenwirbelimplantat zu präparieren, ist es vorteilhaft, wenn die Meißelführung jeweils eine Längsnut auf jeder Anlagefläche umfasst.

Gemäß einer bevorzugten Ausführungsform der Erfindung kann vorgesehen sein, dass der mindestens eine Anschlag am Implantatkörper parallel oder im Wesentlichen parallel zur Längsachse verschiebbar gelagert ist. Dadurch ist es möglich, eine Position des Implantatkörpers relativ zu den benachbart des Zwischenwirbelraums angeordneten Wirbelkörpern parallel oder im Wesentlichen parallel zur Längsachse zu verändern.

Damit der Anschlag auf einfache Weise bewegt werden kann, ist es günstig, wenn ein Antriebsmechanismus vorgesehen ist zum Bewegen des mindestens einen Anschlags, und wenn der Antriebsmechanismus am Schaft oder am Implantatkörper angeordnet und derart ausgebildet ist, dass der zumindestens eine Anschlag in Folge einer Bewegung des Kraftübertragungsglieds bewegbar ist. Beispielsweise kann der Antriebsmechanismus so vorgesehen sein, dass der Anschlag in Folge einer Translations- und/oder einer Rotationsbewegung des Kraftübertragungsglieds bewegbar ist. Der Antriebsmechanismus ermöglicht es also, den Anschlag nur durch Bewegen des Kraftübertragungsglieds, beispielsweise auch über das Betätigungsglied, zu bewegen.

Ein besonders einfacher Aufbau des Führungsinstruments ergibt sich, wenn der Antriebsmechanismus einen Spindeltrieb zum Bewegen des mindestens einen Anschlags umfasst.

Günstig ist es, wenn der Spindeltrieb einen parallel oder im Wesentlichen parallel zu Längsachse am Implantatkörper verschiebbar gelagerten Lagerschlitten, der den mindestens einen Anschlag trägt, und eine rotierbar um eine parallel oder im Wesentlichen parallel zur Längsachse verlaufende Rotationsachse gelagerte Gewindespindel umfasst, die ein zu einem am Lagerschlitten vorgesehenen Innengewinde korrespondierendes Außengewinde aufweist, und wenn die Gewindespindel mit dem Kraftübertragungsglied verbunden oder verbindbar ist. Ein derartig aufgebauter Spindeltrieb ermöglicht es, aufgrund einer Rotation des Kraftübertragungsglieds die Gewindespindel in Rotation zu versetzen und dadurch eine Bewegung des Lagerschlittens parallel oder im Wesentlichen parallel zur Längsachse zu bewirken. Dadurch kann der mindestens eine Anschlag in gewünschter Weise bewegt werden.

Vorzugsweise steht der mindestens eine Anschlag quer zur Längsachse von einem proximalen Ende des Lagerschlittens ab. Auf diese Weise ist es möglich, dass der Anschlag mit dem an den Zwischenwirbelraum angrenzenden Wirbelkörpern in Kontakt treten und so eine Einführtiefe des Implantatkörpers in den Zwischenwirbelraum hinein begrenzen kann.

Damit das Führungsinstrument auf einfache Weise zerlegt werden kann, ist es vorteilhaft, wenn der Antriebsmechanismus eine Kraftübertragungsgliedaufnahme aufweist, in die ein distales Ende des Kraftübertragungsglieds formschlüssig eintaucht. Dies erleichtert zudem, das Instrument herzustellen, insbesondere zu montieren und zu reinigen.

Grundsätzlich wäre es möglich, dass der mindestens eine Anschlag in distaler Richtung weisend vom Implantatkörper abstehend gelagert ist. Allerdings wird dann das Einführen des Implantatkörpers in den Zwischenwirbelraum erschwert. Günstigerweise ist daher der mindestens eine Anschlag in proximaler Richtung weisend vom Implantatkörper abstehend gelagert. Dadurch kann der Implantatkörper in den Zwischenwirbelraum eingeschoben werden, bis der mindestens eine Anschlag an den an den Zwischenwirbelraum angrenzenden Wirbelkörpern anschlägt.

Damit möglichst geringe Kräfte auf die benachbarten Wirbelkörper einwirken, wenn der mindestens eine Anschlag an ihnen anliegt um eine Einführtiefe des Implantatkörpers in den Zwischenwirbelraum zu begrenzen, ist es vorteilhaft, wenn zwei oder vier Anschläge vorgesehen sind. Dadurch wird eine Beschädigung der Wirbelkörper in unerwünschter Weise vermieden.

Besonders einfach wird der Aufbau des Führungsinstruments, wenn es spiegelsymmetrisch zu einer die Längsachse enthaltenden Spiegelebene ausgebildet ist. Beispielsweise kann es auch symmetrisch zu zwei zueinander senkrecht stehenden Spiegelebenen ausgebildet sein.

Die eingangs gestellte Aufgabe wird ferner gelöst durch einen Satz chirurgischer Führungsinstrumente für ein chirurgisches Bearbeitungswerkzeug mit einem Implantatkörper zum Einführen in einen Zwischenwirbelraum zwischen benachbarte Wirbelkörper einer menschlichen oder tierischen Wirbelsäule, mit einem Schaft und mit einer Anschlagvorrichtung zum Begrenzen einer Einführtiefe des Implantatkörpers in den Zwischenwirbelraum, wobei der Schaft ein distales und ein proximales Ende aufweist, wobei der Implantatkörper zwei an den benachbarten Wirbelkörpern anlegbare Anliegeflächen definiert und am distalen Ende des Schafts angeordnet ist, wobei der Satz zwei oder mehr der oben beschriebenen Führungsinstrumente umfasst und wobei die zwei oder mehr Führungsinstrumente unterschiedlich große und/oder unterschiedlich geformte Implantatkörper aufweisen. Mit einem derartigen Satz ist es möglich, im Falle eines chirurgischen Eingriffs jeweils das optimal in Form und Größe geeignete Führungsinstrument auszuwählen, um es vor der Bearbeitung der Wirbelkörperflächen mit dem Bearbeitungswerkzeug in den Zwischenwirbelraum einzuführen.

Da der Implantatkörper insbesondere so gewählt wird, dass er den Zwischenwirbelraum derart ausfüllt, dass ein Abstand der benachbarten Wirbelkörper auch während des chirurgischen Eingriffs dem ursprünglichen Abstand derselben entspricht, ist es günstig, wenn die Implantatkörper unterschiedliche Dicken und/oder unterschiedliche Neigungswinkel zwischen den Anlageflächen aufweisen. Dadurch werden weitere Beschädigungen der Wirbelsäule, insbesondere der an den Zwischenwirbelraum angrenzenden Wirbelkörper, vermieden.

Die eingangs gestellte Aufgabe wird ferner erfindungsgemäß durch ein chirurgisches Instrumentarium gelöst, umfassend mindestens ein chirurgisches Bearbeitungswerkzeug, insbesondere einen Meißel, und mindestens ein chirurgisches Führungsinstrument für das mindestens eine chirurgische Bearbeitungswerkzeug mit einem Implantatkörper zum Einführen in einen Zwischenwirbelraum zwischen benachbarte Wirbelkörper einer menschlichen oder tierischen Wirbelsäule, mit einem Schaft und mit einer Anschlagvorrichtung zum Begrenzen einer Einführtiefe des Führungsinstruments in den Zwischenwirbelraum, wobei der Schaft ein distales und ein proximales Ende aufweist, wobei der Implantatkörper zwei an den benachbarten Wirbelkörpern anlegbare Anlageflächen definiert und am distalen Ende des Schafts angeordnet ist, wobei die Anschlagvorrichtung mindestens einen am distalen Ende des Schafts oder am Implantatkörper beweglich gelagerten Anschlag aufweist, der eine quer oder im Wesentlichen quer zu mindestens einer der Anlageflächen in distaler Richtung weisende Anschlagfläche aufweist, wobei das mindestens eine chirurgische Führungsinstrument eines der oben beschriebenen Führungsinstrumente oder einer der oben beschriebenen Sätze von Führungsinstrumenten ist.

Das erfindungsgemäße Instrumentarium eignet sich hervorragend, um Wirbelkörper mit Aufnahmen zu versehen, in die ein Vorsprung an einem Zwischenwirbelimplantat eintauchen kann. Insbesondere wird mit dem Instrumentarium vermieden, dass das Bearbeitungswerkzeug zu tief in anteriorer/posteriorer Richtung in den Zwischenwirbelraum eingeführt werden kann.

Grundsätzlich wäre es denkbar, dass das mindestens eine Bearbeitungswerkzeug ein Fräswerkzeug ist, mit dem eine Nut oder eine Ausnehmung in einen Wirbelkörper gefräst werden kann. Vorzugsweise ist das mindestens eine Bearbeitungswerkzeug jedoch ein Einfach- oder ein Doppelmeißel. Damit lässt sich eine Nut auf einfache Weise in den jeweiligen Wirbelkörper treiben.

Günstig ist es, wenn das mindestens eine Bearbeitungswerkzeug einen hohlen Werkzeugschaft aufweist, an dessen distalem Ende mindestens eine Werkzeugschneide angeordnet ist und wenn der Schaft des Führungsinstruments in den Werkzeugschaft einführbar ist. Beispielsweise bei einem Führungsinstrument, bei dem sich der Griffteil vom Schaft trennen lässt, kann nach Einführen des Implantatkörpers in den Zwischenwirbelraum beispielsweise das Griffteil entfernt und das Bearbeitungswerkzeug mit seinem Werkzeugschaft über den Schaft des Führungsinstruments vorgeschoben werden. Dadurch wird das Beabeitungswerkzeug zusätzlich am Führungsinstrument geführt.

Die eingangs gestellte Aufgabe wird ferner auch erfindungsgemäß durch ein Verfahren zum Vorbereiten eines Zwischenwirbelraums zum Einsetzen eines Zwischenwirbelimplantats mit zwei zur Anlage an an den Zwischenwirbelraum angrenzenden Wirbelkörperflächen benachbarter Wirbelkörper einer menschlichen oder tierischen Wirbelsäule ausgebildeten, im Wesentlichen voneinander weg weisenden Implantatanlageflächen gelöst, welche mindestens einen quer oder im Wesentlichen quer von mindestens einer Implantatanlagefläche abstehenden finnenartigen Vorsprung aufweisen, bei welchem der Zwischenwirbelraum ausgeräumt wird, bei welchem ein chirurgisches Führungsinstrument mit einem Implantatkörper, einer Führung für ein Bearbeitungswerkzeug und einer mindestens einen Anschlag umfassenden Anschlagvorrichtung verwendet wird, wobei der mindestens eine Anschlag so eingestellt wird, dass der Implantatkörper nur minimal tief in den Zwischenwirbelraum eingeführt werden kann, wobei der Implantatkörper in den Zwischenwirbelraum eingeführt wird bis der mindestens eine Anschlag an den benachbarten Wirbelkörpern anschlägt, wobei der mindestens eine Anschlag in Abhängigkeit einer gewünschten Werkzeugtiefe relativ zum Implantatkörper verstellt wird und der Implantatkörper weiter in den Zwischenwirbelraum getrieben wird, bis der zumindestens eine Anschlag wieder an den benachbarten Wirbelkörpern anschlägt, und wobei mit dem Bearbeitungswerkzeug, das in der Führung geführt wird, mindestens eine Vertiefung in mindestens einen der beiden benachbarten Wirbelkörper eingearbeitet wird.

Das erfindungsgemäße Verfahren stellt sicher, dass die Vertiefung derart in mindestens einen Wirbelkörper eingearbeitet wird, dass darüber hinaus eine Beschädigung des Wirbelkörpers und auch eine weitergehende Verletzung der Wirbelsäule, beispielsweise des Spinalkanals, vermieden wird. Beispielsweise kann als Bearbeitungswerkzeug ein Meißel verwendet werden, der in der Führung geführt und mit dem die Vertiefung in den Wirbelkörper eingeschlagen wird.

Vorteilhafterweise wird als Führungsinstrument eines der oben beschriebenen Führungsinstrumente verwendet. Das Verfahren mit einem solchen Führungsinstrument durchzuführen hat den Vorteil, dass eine unbeabsichtigte Verletzung der Wirbelsäule durch den chirurgischen Eingriff vermieden wird.

Um zu verhindern, dass das Bearbeitungswerkzeug zu weit in den Wirbelkörper vorgetrieben werden kann, ist es vorteilhaft, wenn der mindestens eine Anschlag unter Röntgenkontrolle verstellt wird. So wird sichergestellt, dass weder der Implantatkörper noch das in der Führung geführte Bearbeitungswerkzeug die Wirbelsäule in unerwünschter Weise verletzen können.

Günstig ist es ferner, wenn eines der oben beschriebenen Instrumentarien verwendet wird.

Um bei der Präparation des mindestens einen Wirbelkörpers sicherzustellen, dass das einzusetzende Zwischenwirbelimplantat optimal in den Zwischenwirbelraum eingepasst werden kann, ist es vorteilhaft, wenn ein Führungsinstrument mit einem Implantatkörper ausgewählt wird, und wenn eine Höhe des Implantatkörpers und ein Winkel zwischen den Implantatanlageflächen des Implantatkörpers einer Höhe und einem Winkel eines zu implantierenden Implantats entsprechen. Ein Operateur kann also, wenn feststeht, welches Implantat er in den Zwischenwirbelraum einsetzen möchte, das entsprechende Führungsinstrument auswählen, was insbesondere den Vorteil hat, dass er mit dem Bearbeitungswerkzeug ohne weiteres Nachdenken die Vertiefung in den jeweiligen Wirbelkörper einarbeiten kann und dabei nicht fürchten muss, die Wirbelsäule in unerwünschter Weise zu verletzen.

Die nachfolgende Beschreibung einer bevorzugten Ausführungsform der Erfindung dient im Zusammenhang mit der Zeichnung der näheren Erläuterung. Es zeigen:
- Figur 1 :: eine perspektivische Darstellung eines erfindungsgemäßen Führungsinstruments;
- Figur 2:: eine teilweise geschnittene Ansicht eines distalen Endes des Instruments aus Figur 1 längs Linie 2-2;
- Figur 3:: eine schematische Darstellung bei der Bearbeitung eines Wirbelkörpers mit einem erfindungsgemäßen Instrumentarium; und

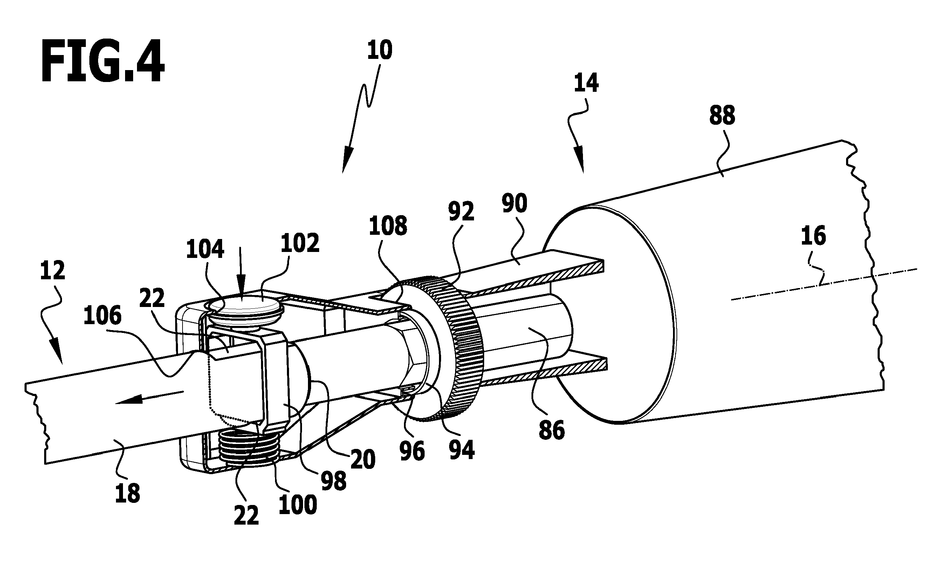

- Figur 4:: eine Schnittansicht längs Linie 4-4 in Figur 1.

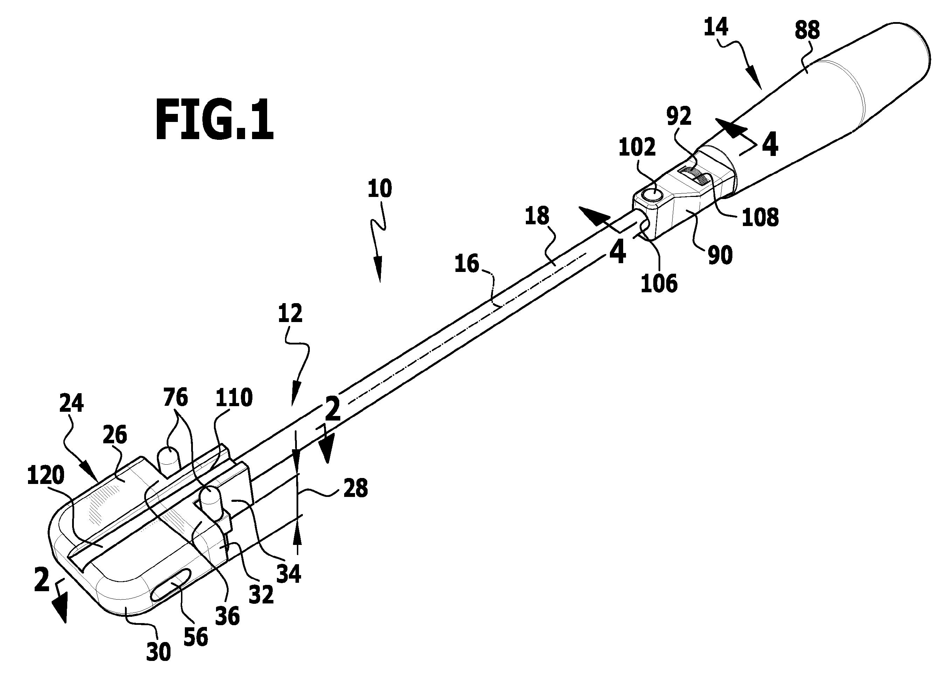

In Figur 1 ist ein insgesamt mit dem Bezugszeichen 10 versehenes chirurgisches Führungsinstrument dargestellt, welches im Wesentlichen zwei Teile umfasst, nämlich einen Schaftteil 12 und einen Griffteil 14, die miteinander lösbar verbindbar sind.

Der Schaftteil 12 umfasst einen langgestreckten, eine Längsachse 16 definierenden hohlen Schaft 18, welcher benachbart einem proximalen Ende 20 mit zwei diametral gegenüberliegenden Abflachungen 22 versehen ist.

An einem distalen Ende des Schafts 18 ist ein Implantatkörper 24 angeordnet, welcher im Wesentlichen quaderförmig ausgebildet ist. Er umfasst zwei in entgegengesetzte Richtungen weisende Anlageflächen, die relativ zueinander um einen Neigungswinkel 28 geneigt sind. Je nach Einsatzzweck des Führungsinstruments 10 kann der Neigungswinkel 28 Werte in einem Bereich von 0° bis 30° annehmen. Der Implantatkörper 24 ist zweiteilig aufgebaut und umfasst einen Vorderteil 30 und einen Hinterteil 32, wobei der Hinterteil 32 im Wesentlichen T-förmig ausgebildet ist. Der Hinterteil 32 umfasst einen Mittelkörper 34, welcher sich im Wesentlichen parallel zur Längsachse 16 erstreckt, und zwei an einem distalen Ende quer abstehende Seitenkörper 36, die an eine proximale Endfläche des Vorderteils 30 bündig anschließen. Die Seitenkörper 36 sind mit parallel zur Längsachse 16 verlaufenden Bohrungen 38 versehen, die koaxial mit einem Innengewinde 40 versehenen Bohrungen 42 am Vorderteil 30 ausgerichtet sind. Mittels zweier Schrauben 44, die einen mit einem zum Innengewinde 40 korrespondieren Außengewinde 46 versehenen Schaft aufweisen, der durch die Bohrungen 38 einführbar ist, ist der Hinterteil mit dem Vorderteil 30 verschraubt.

Der Mittelkörper 34 ist koaxial zur Längsachse 16 mit einer Längsbohrung 48 versehen, die sich in etwa auf der halben Länge des Mittelkörpers 34 einstufig verjüngt und proximalseitig eine Schaftaufnahme 50 bildet, in die das distale Ende 52 des Schafts einführbar und am Mittelkörper 34 festlegbar ist, beispielsweise durch Schrauben, Schweißen oder Kleben.

Der Vorderteil 30 ist mit einer im Wesentlichen quaderförmigen Ausnehmung 54 versehen, die in proximaler Richtung weisend offen, allerdings durch das Hinterteil 32 verschlossen ist. Die Ausnehmung 54 ist ferner quer zur Längsachse 16 mit zwei Fenstern in Form von Langlöchern 56 versehen. Die Ausnehmung 54 dient zur Aufnahme und rotierbaren Lagerung einer koaxial zur Längsachse 16 zwischen einer distalseitigen Begrenzung der Ausnehmung 54 und dem distalen Ende des Hinterteils 32 angeordneten Gewindespindel 58, in welcher ein in proximaler Richtung weisend geöffneter Innenachtkant 60 ausgebildet ist, der eine Wellenaufnahme bildet. In der Ausnehmung 54 ist auf der Gewindespindel 58 ein Lagerschlitten 62 gelagert, welcher einen sich quer zur Längsachse 16 erstreckenden Querträger 64 umfasst, der mit einer koaxial zur Längsachse 16 verlaufenden und mit einem zum Außengewinde der Gewindespindel 58 korrespondierenden Innengewinde 66 versehen ist. Parallel zur Längsachse 16 sind am Querträger 64 symmetrisch zu dieser Bohrungen 68 vorgesehen, in die Führungsstäbe 70 eingesetzt und fest mit dem Querträger 64 verbunden sind. Parallel zu den Bohrungen 38 sind an den Seitenkörpern 36 zwei Führungsbohrungen 72 vorgesehen, die von den Führungsstäben 70 durchsetzt werden. Proximale Enden der Führungsstäbe 70 enden in einem würfelförmigen Führungskörper 74, dessen Kantenlänge größer ist als ein Durchmesser der Führungsstäbe 70. Quer zur Längsachse 16 und im Wesentlichen quer zu den Anlageflächen 26 stehen von jedem Führungskörper 74 zwei Anschläge bildende Anschlagzapfen 76 ab, deren freie Enden halbkugelig abgerundet sind und deren in distaler Richtung weisende Seitenflächen Anschlagflächen 77 bilden.

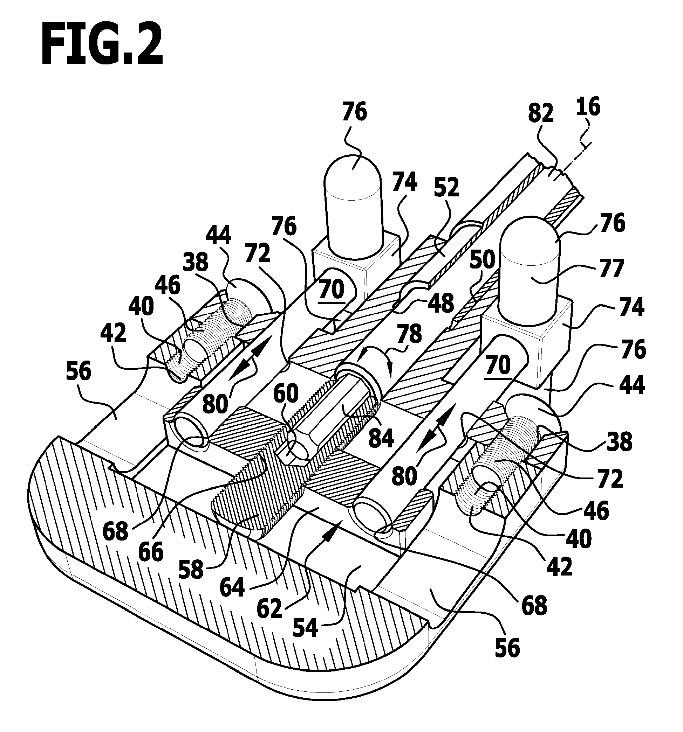

In Folge einer Rotation der Gewindespindel 58, wie in Figur 2 durch den Pfeil 78 symbolisiert, wird der Lagerschlitten 62 in einer Richtung parallel zur Längsachse 16 entweder in distaler oder proximaler Richtung bewegt, was durch die Pfeile 80 in Figur 2 symbolisiert ist.

Zum Antreiben der Gewindespindel 58 ist eine, ein Kraftübertragungsglied bildende Antriebswelle 82 vorgesehen, die den Schaft 18 durchsetzt und an ihrem distalen Ende mit einem zum Innenachtkant 60 korrespondierenden Achtkant 84 versehen ist, der von proximal her kommend in den Innenachtkant 60 formschlüssig einführbar ist. Ein proximales Ende der Antriebswelle 82 ist in Form eines Sechskant 86 geformt.

Der Griffteil 14 umfasst einen langgestreckten, ergonomisch geformten Handgriff 88, an den sich distalseitig ein Kupplungsgehäuse 90 anschließt. Am Gehäuse 90 ist rotierbar um die Längsachse 16 ein Antriebsrad 92 gelagert, welches ein Betätigungsglied bildet. Es ist auf einer Außenseite mit einer flachen Verzahnung versehen, die ein Betätigen des Antriebsrads 92 erleichtert. Koaxial zur Längsachse 16 ist das Antriebsrad 92 mit einem Innensechskant versehen, der korrespondierend zum Sechskant 86 ausgebildet ist, so dass dieser formschlüssig durch das Antriebsrad 92 hindurchgesteckt werden kann. Ferner weist das Antriebsrad 92 distalseitig einen Ringflansch 94 auf, der in eine korrespondierende, am Kupplungsgehäuse 90 in proximaler Richtung hin geöffnete Ringnut 96 eintaucht und in dieser geführt ist, so dass das Antriebsrad 92 um die Längsachse 16 verdrehbar am Kupplungsgehäuse 90 gelagert ist. Der Sechskant 86 und der nicht dargestellte Innensechskant des Antriebsrads 92 bilden jeweils ein Kupplungsglied einer Kupplungsvorrichtung, mit der die Antriebswelle 82 mit dem Antriebsrad 92 lösbar verbindbar ist.

Im Bereich des distalen Endes des Kupplungsgehäuses 90 ist ein quadratischer Rahmen 98 quer zur Längsachse verschiebbar gelagert, und zwar stützt er sich an einer Seite am Gehäuse 90 über eine Schraubenfeder 100 ab und ist auf einer gegenüberliegenden Seite mit einem Druckknopf 102 verbunden, der in einer Ausnehmung 104 des Kupplungsgehäuses 90 geführt ist. Durch die Schraubenfeder 100 wird der Rahmen 98 in Richtung auf den Druckknopf 102 hin gedrückt, der in einer Grundstellung am Kupplungsgehäuse 90 anschlägt. Die Abflachungen 22 und der Rahmen 98 bilden Verbindungsglieder einer Verbindungsvorrichtung, und zwar in Form einer Rastverbindung. Die Abflachungen 22 bilden Rastglieder in Form von Rastausnehmungen und der Rahmen 98 bildet ein Rastglied in Form einer am Griffteil 14 federnd vorgespannt gelagerten Rastklinke.

Distalseitig ist am Kupplungsgehäuse 90 eine kreisförmige Öffnung 106 vorgesehen, deren Durchmesser korrespondierend zu einem Außendurchmesser des Schafts 18 gewählt ist. Ferner sind am Kupplungsgehäuse 90 Querschlitze 108 vorgesehen, durch die ein Teil des Antriebsrads 92 aus dem Kupplungsgehäuse 90 hervorsteht.

Zum Verbinden des Schaftteils 12 mit dem Griffteil 14 wird zunächst die proximalseitig aus dem Schaft 18 vorstehende Antriebswelle 82 durch die Öffnung 106, durch den Rahmen 98 und durch das Antriebsrad 92 durchgesteckt. Gleichzeitig wird das proximale Ende des Schafts 18 durch die Öffnung 106 und den Rahmen 98 durchgesteckt. Beim Einführen gleitet der Rahmen 98 am Ende 20 des Schafts 18 auf und wird gegen die Federkraft der Schraubenfeder 100 in Richtung auf diese bewegt. Sobald der Schaft 18 so weit in das Kupplungsgehäuse 90 eingeführt ist, dass der Rahmen 98 in die Abflachung 22 eintauchen kann, drückt die Schraubenfeder 100 den Rahmen 98 in Richtung auf den Schaft 18 und damit auch den Druckknopf 102 gegen das Kupplungsgehäuse 90. Auf diese Weise sind der Schaft 18 und die Antriebswelle 82 axial am Griffteil 14 gesichert.

Am Implantatkörper 24 sind zwei Längsnuten 110 in den Anlagenflächen 26 vorgesehen und erstrecken sich parallel zur Längsachse 16. Ein distales Ende der Längsnut 110 endet mit einem distalen Ende des Vorderteils 30, ein proximales Ende der Längsnut 110 endet mit einem proximalen Ende des Mittelkörpers 34. Die Längsnuten 110 bilden Führungsnuten für ein chirurgisches Bearbeitungswerkzeug, beispielsweise einen chirurgischen Meißel 112.

Das Führungsinstrument 110 kann Teil eines Satzes von Führungsinstrumenten sein. Ein solcher Satz kann beispielsweise ein Griffteil 14 umfassen und eine Mehrzahl von Schaftteilen 12. Die Schaftteile 12 unterscheiden sich vorzugsweise in Form und Größe des Implantatkörpers 24, insbesondere weisen diese Implantatkörper 24 unterschiedliche Abstände der Anlageflächen 26 voneinander sowie unterschiedliche Neigungswinkel 28 auf.

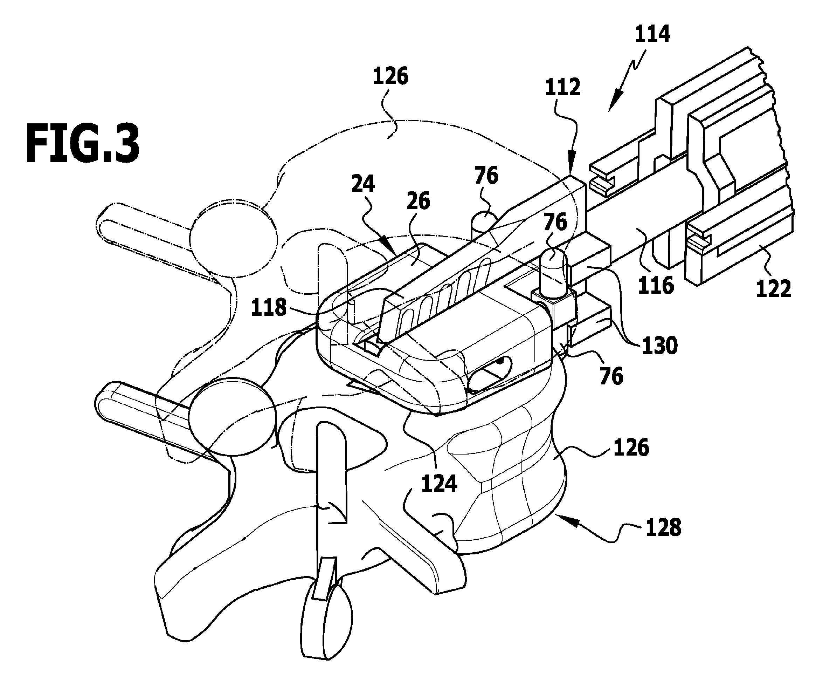

Ferner kann das Führungsinstrument 10 auch Teil eines chirurgischen Instrumentariums sein, welches insbesondere mindestens einen Meißel 112 umfasst. Der Meißel 112 umfasst vorzugsweise einen hohlen Schaft 116, der nach Lösen des Griffteils 14 vom Schaftteil 12 über den Schaft 18 in distaler Richtung geschoben werden kann. An einem distalen Ende des Schafts 116 ist, wie beispielsweise in Figur 3 dargestellt, eine zweiteilige Schneide 118 angeordnet, so dass insgesamt ein so genannter Doppelmeißel ausgebildet wird. Die Schneide 118 ist so ausgebildet, dass ihre Breite einer Breite der Längsnuten 110 entspricht und zudem ein Abstand der beiden die Schneide 118 bildenden Schneidenhälften voneinander einem Abstand von Nutböden 120 der Längsnuten 110 quer zur Längsachse 16 entspricht. Ferner kann der Meißel 112 einen am Schaft 116 gelagerten Klingenschutz 122 umfassen, welcher in einer Grundstellung federnd vorgespannt über der Schneide 118 gehalten ist und entgegen der Kraft einer nicht dargestellten Feder in proximaler Richtung zurückgezogen werden kann.

Der Einsatz des Instrumentariums 114 wird nachfolgend näher beschrieben. Zur Vorbereitung des Führungsinstruments 10 wird der Schaftteil 12 mit dem Griffteil 14 in der oben beschriebenen Weise verbunden. Durch Drehen des Antriebsrads 92 wird die Antriebswelle 82 in Rotation versetzt, und zwar so lange, bis der Lagerschlitten 62 in seine distalste Position gebracht ist und die Führungskörper 74 an den Seitenkörpern 36 anschlagen. Die Anschlagzapfen 76 nehmen jetzt ihre distalste Stellung bezogen auf den Schaft 18 ein.

Der Implantatkörper 24 wird durch einen Zugang in einen menschlichen oder tierischen Körper und in einen Zwischenwirbelraum 124 zwischen benachbarten Wirbelkörpern 126 einer Wirbelsäule 128 eingeführt. Der Schaftteil 12 wird dabei so ausgewählt, dass der Implantatkörper 24 einem später in den Zwischenwirbelraum 124 zu implantierenden Implantat insoweit entspricht, als der Abstand der Anlageflächen 26 und eine Neigung derselben relativ zueinander übereinstimmen. Der Implantatkörper 24 wird so weit in den Zwischenwirbelraum 124 eingeführt, bis die über die Anlageflächen 26 vorstehenden Anschlagzapfen 76 an den Wirbelkörpern 126 anschlagen. Beispielsweise unter Röntgenkontrolle kann ein Operateur nun feststellen, ob der Implantatkörper 24 weit genug in den Zwischenwirbelraum 124 vorgeschoben wurde. Ist dies nicht der Fall, dann wird mit dem Antriebsrad 92 die Antriebswelle 82 wieder in Rotation versetzt, jetzt allerdings in entgegengesetzter Richtung wie zur Vorbereitung des Führungsinstruments 10, um die Führungskörper 74 von den Seitenkörpern 36 wegzubewegen. Die Anschlagzapfen 76 werden so in proximaler Richtung und vom Implantatkörper 24 weg bewegt, und zwar gerade so weit, wie der Implantatkörper 24 noch weiter in den Zwischenwirbelraum 124 eingeführt werden darf. Nach Verstellen der Anschlagzapfen 76 wird der Implantatkörper 24 weiter in den Zwischenwirbelraum 124 vorgetrieben, bis die Anschlagzapfen 76 wieder an den Wirbelkörpern 126 anschlagen. Dieser Justiervorgang wird so lange fortgesetzt, bis der Implantatkörper 24 die gewünschte Position im Zwischenwirbelraum 124 einnimmt.

Zum Einarbeiten einer Ausnehmung in Oberflächen der Wirbelkörper 126, insbesondere zur Aufnahme beispielsweise eines finnenartigen Vorsprungs an dem später einzusetzenden Zwischenwirbelimplantat, wird der Griffteil 14 vom Schaftteil 12 getrennt, indem durch Drücken auf den Druckknopf 102 die Schraubenfeder 100 etwas zusammengedrückt wird, so dass der Rahmen 98 die Abflachung 22 freigibt und der Schaft 18 in distaler Richtung aus dem Kupplungsgehäuse 90 herausgezogen werden kann. Anschließend wird der Meißel 112 mit der Schneide 118 in gewünschter Form und Größe mit seinem Schaft 116 über den Schaft 18 geschoben. An der Schneide 118 quer abstehende Vorsprünge 130 bilden Anschläge, die verhindern, dass die Schneide 118 zu weit in distaler Richtung vorgetrieben werden kann. Sie ist so ausgebildet, dass sie an den Anschlagzapfen 76 oder alternativ auch am proximalen Ende des Mittelkörpers 34 anschlagen kann.

Durch das Führungsinstrument 10 und der an diesem bewegbar gelagerten Anschlagzapfen 76 wird verhindert, dass der Meißel 112 zu tief in den Zwischenwirbelraum 124 eingetrieben werden kann, was zu einer unnötigen Teilresektion der Wirbelkörper 126 führen und im schlimmsten Fall sogar eine Verletzung der im Spinalkanal verlaufenden Nerven bedeuten würde.

Sind die Nuten in den Wirbelkörpern 126 in gewünschter Weise ausgebildet, wird zunächst der Meißel 112 vom Schaftteil 12 abgezogen, anschließend der Griffteil 14 wieder mit dem Schaftteil 12 verbunden und dann das Führungsinstrument 10 aus dem Körper entfernt.

Das Führungsinstrument 10 sowie auch das gesamte Instrumentarium 114 sind aus Materialien hergestellt, die dampfsterilisierbar sind. Vorzugsweise ist das gesamte Führungsinstrument 10, bis auf den Handgriff 88, aus einem Instrumentenstahl hergestellt.

## Patentansprüche

1. Chirurgisches Führungsinstrument (10) für ein chirurgisches Bearbeitungswerkzeug (112), insbesondere einen Meißel (112), mit einem Implantatkörper (24) zum Einführen in einen Zwischenwirbelraum (124) zwischen benachbarte Wirbelkörper (126) einer menschlichen oder tierischen Wirbelsäule (128), mit einem Schaft (18) und mit einer Anschlagvorrichtung (76) zum Begrenzen einer Einführtiefe des Implantatkörpers (24) in den Zwischenwirbelraum (124), wobei der Schaft (18) ein distales und ein proximales Ende (20, 52) aufweist, wobei der Implantatkörper (24) zwei an den benachbarten Wirbelkörpern (126) anlegbare Anlageflächen (26) definiert und am distalen Ende des Schafts (18) angeordnet ist, wobei die Anschlagvorrichtung (76) mindestens einen am distalen Ende des Schafts (18) oder am Implantatkörper (24) beweglich gelagerten Anschlag (76) aufweist, der eine quer oder im Wesentlichen quer zu mindestens einer der Anlageflächen (26) in distaler Richtung weisende Anschlagfläche (77) aufweist, **dadurch gekennzeichnet, dass** die Anschlagvorrichtung (76) ein am proximalen Ende oder im Bereich des proximalen Endes des Schafts (18) angeordnetes Betätigungsglied (92) und ein am Schaft (18) gelagertes Kraftübertragungsglied (82) umfasst und dass das Betätigungsglied (92), das Kraftübertragungsglied (82) und der mindestens eine Anschlag (76) derart angeordnet und zusammenwirkend ausgebildet sind, dass durch Betätigung des Betätigungsglieds (92) der mindestens eine Anschlag (76) in proximaler und/oder distaler Richtung bewegbar ist.

2. Führungsinstrument nach Anspruch 1, **dadurch gekennzeichnet, dass** das Betätigungsglied (92) mit dem Kraftübertragungsglied (82) lösbar verbindbar ist.

3. Führungsinstrument nach Anspruch 2, **dadurch gekennzeichnet, dass** eine Kupplungsvorrichtung vorgesehen ist zum Verbinden des Kraftübertragungsglieds (82) mit dem Betätigungsglied (92), dass ein erstes und ein mit diesem in einer Kupplungsstellung in Eingriff bringbares zweites Kupplungsglied vorgesehen sind, dass das erste Kupplungsglied am Betätigungsglied (82) angeordnet ist und dass das zweite Kupplungsglied (86) am Kraftübertragungsglied (82) angeordnet ist.

4. Führungsinstrument nach Anspruch 3, **dadurch gekennzeichnet, dass** das eine der beiden Kupplungsglieder ein Außenvielkant (86) oder ein Außenvielrund ist und dass das andere der beiden Kupplungsglieder ein korrespondierender Innenvielkant oder ein Innenvielrund ist.

5. Führungsinstrument nach einem der voranstehenden Ansprüche, **dadurch gekennzeichnet, dass** am proximalen Ende des Schafts (18) ein Griffteil (14) vorgesehen ist.

6. Führungsinstrument nach Anspruch 5, **dadurch gekennzeichnet, dass** das Griffteil (14) mit dem Schaft (18) lösbar verbindbar ist.

7. Führungsinstrument nach Anspruch 6, **dadurch gekennzeichnet, dass** eine Verbindungsvorrichtung vorgesehen ist zum Verbinden des Schafts (18) m it dem Griffteil (14), dass ein erstes und ein m it diesem in einer Verbindungsstellung in Eingriff bringbares zweites Verbindungsglied (22, 98) vorgesehen ist und dass das erste Verbindungsglied (22) am Schaft (18) angeordnet ist und dass das zweite Verbindungsglied (98) am Griffteil (14) angeordnet ist.

8. Führungsinstrument nach Anspruch 7, **dadurch gekennzeichnet, dass** das eine der beiden Verbindungsglieder (22, 98) ein Außenvielkant (22) oder ein Außenvielrund ist und dass das andere der beiden Verbindungsglieder (22, 98) ein korrespondierender Innenvielkant oder ein Innenvielrund ist.

9. Führungsinstrument nach Anspruch 8, **dadurch gekennzeichnet, dass** der Außenvielkant (22) einen kurzen Schaftabschnitt des Schafts (18) bildet und dass der Innenvielkant am Griffteil (14) angeordnet ist.

10. Führungsinstrument nach einem der Ansprüche 7 bis 9, **dadurch gekennzeichnet, dass** die Verbindungsvorrichtung (22, 98) in Form einer Rastverbindung ausgebildet ist, dass die beiden Verbindungsglieder (22, 98) in Form von in einer Raststellung in Eingriff stehenden Rastglieder (22, 98) ausgebildet sind und dass die beiden Rastglieder derart angeordnet sind, dass sie relativ zueinander bewegbar sind beim Übergang von der Raststellung in eine Lösestellung, in der sie außer Eingriff stehen, und umgekehrt.

11. Führungsinstrument nach Anspruch 10, **dadurch gekennzeichnet, dass** eines der Rastglieder (22, 98) in Form einer am Schaft (18) angeordneten Rastausnehmung (22) ausgebildet ist und dass das andere Rastglied (98) eine federnd vorgespannte, am Griffteil (14) gelagerte Rastklinke (98) ist.

12. Führungsinstrument nach Anspruch 11, **dadurch gekennzeichnet, dass** die die Rastklinke (98) in einer Richtung quer zur Längsachse (16) des Schafts (18) bewegbar gelagert ist.

13. Führungsinstrument nach einem der Ansprüche 5 bis 12, **dadurch gekennzeichnet, dass** das Betätigungsglied (92) am Griffteil (14) angeordnet ist.

14. Führungsinstrument nach einem der voranstehenden Ansprüche, **dadurch gekennzeichnet, dass** das Kraftübertragungsglied (82) eine am Schaft (18) rotierbar gelagerte Antriebswelle (82) ist.

15. Führungsinstrument nach einem der voranstehenden Ansprüche, **dadurch gekennzeichnet, dass** das Betätigungsglied (92) ein um eine Längsachse (16) des Schafts (18) rotierbar gelagertes Antriebsrad (92) ist.

16. Führungsinstrument nach einem der voranstehenden Ansprüche, **dadurch gekennzeichnet, dass** am Implantatkörper (24) eine Meißelführung (110) vorgesehen ist.

17. Führungsinstrument nach Anspruch 16, **dadurch gekennzeichnet, dass** die Meißelführung (110) mindestens eine Führungsnut (110) umfasst und dass die mindestens eine Führungsnut (110) in einer der beiden Anlageflächen (26) parallel oder im Wesentlichen parallel zur Längsachse (16) verlaufend angeordnet ist.

18. Führungsinstrument nach einem der Ansprüche 16 oder 17, **dadurch gekennzeichnet, dass** mindestens ein Teil der Meißelführung (110) proximalseitig vom Implantatkörper (24) vorsteht.

19. Führungsinstrument nach einem der Ansprüche 16 bis 18, **dadurch gekennzeichnet, dass** die Meißelführung (110) jeweils eine Längsnut (110) auf jeder Anlagefläche (26) umfasst.

20. Führungsinstrument nach einem der voranstehenden Ansprüche, **dadurch gekennzeichnet, dass** der mindestens eine Anschlag (76) am Implantatkörper (24) parallel oder im Wesentlichen parallel zur Längsachse (16) verschiebbar gelagert ist.

21. Führungsinstrument nach einem der voranstehenden Ansprüche, **dadurch gekennzeichnet, dass** ein Antriebsmechanismus vorgesehen ist zum Bewegen des mindestens einen Anschlags (76), dass der Antriebsmechanismus (58, 62) am Schaft (18) oder am Implantatkörper (24) angeordnet und derart ausgebildet ist und dass der mindestens eine Anschlag (76) in Folge einer Rotationsbewegung des Kraftübertragungsglieds (82) bewegbar ist.

22. Führungsinstrument nach Anspruch 21, **dadurch gekennzeichnet, dass** der Antriebsmechanismus (58, 62) einen Spindeltrieb (58, 62) zum Bewegen des mindestens einen Anschlags (76) umfasst.

23. Führungsinstrument nach Anspruch 22, **dadurch gekennzeichnet, dass** der Spindeltrieb (58, 62) einen parallel oder im Wesentlichen parallel zur Längsachse (16) am Implantatkörper (24) verschiebbar gelagerten Lagerschlitten (62), der den mindestens einen Anschlag (76) trägt, und eine rotierbar um eine parallel oder im Wesentlichen parallel zur Längsachse (16) verlaufende Rotationsachse gelagerte Gewindespindel (58) umfasst, die ein zu einem am Lagerschlitten (62) vorgesehenen Innengewinde (66) korrespondierendes Außengewinde aufweist, und dass die Gewindespindel (58) mit dem Kraftübertragungsglied (82) verbunden oder verbindbar ist.

24. Führungsinstrument nach einem der Ansprüche 23 oder 24, **dadurch gekennzeichnet, dass** der mindestens eine Anschlag (76) quer zur Längsachse (16) von einem proximalen Ende des Lagerschlittens (62) absteht.

25. Führungsinstrument nach einem der Ansprüche 21 bis 24, **dadurch gekennzeichnet, dass** der Antriebsmechanismus (58, 62) eine Kraftübertragungsgliedaufnahme (60) aufweist, in die ein distales Ende (84) des Kraftübertragungsglied (82) formschlüssig eintaucht.

26. Führungsinstrument nach einem der voranstehenden Ansprüche, **dadurch gekennzeichnet, dass** der mindestens eine Anschlag (76) in proximaler Richtung weisend vom Implantatkörper (24) abstehend gelagert ist.

27. Führungsinstrument nach einem der voranstehenden Ansprüche, **dadurch gekennzeichnet, dass** zwei oder vier Anschläge (76) vorgesehen sind.

28. Führungsinstrument nach einem der voranstehenden Ansprüche, **dadurch gekennzeichnet, dass** das Instrument (10) spiegelsymmetrisch zu einer die Längsachse (16) enthaltenden Spiegelebene ausgebildet ist.

29. Satz chirurgischer Führungsinstrumente (10) für ein chirurgisches Bearbeitungswerkzeug (112) mit einem Implantatkörper (24) zum Einführen in einen Zwischenwirbelraum (124) zwischen benachbarte Wirbelkörper (126) einer menschlichen oder tierischen Wirbelsäule (128), mit einem Schaft (18) und mit einer Anschlagvorrichtung (76) zum Begrenzen einer Einführtiefe des Implantatkörpers (24) in den Zwischenwirbelraum (124), wobei der Schaft (18) ein distales und ein proximales Ende (20, 52) aufweist, wobei der Implantatkörper (24) zwei an den benachbarten Wirbelkörpern (126) anlegbare Anlageflächen (26) definiert und am distalen Ende des Schafts (18) angeordnet ist, **dadurch gekennzeichnet, dass** der Satz zwei oder mehr Führungsinstrumente (10) nach einem der voranstehenden Ansprüche umfasst und dass die zwei oder mehr Führungsinstrumente (10) unterschiedlich große und/oder unterschiedlich geformte Implantatkörper (24) aufweisen.

30. Satz nach Anspruch 29, **dadurch gekennzeichnet, dass** die Implantatkörper (24) unterschiedliche Dicken und/oder unterschiedliche Neigungswinkel (28) zwischen den Anlageflächen (26) aufweisen.

31. Chirurgisches Instrumentarium (114) umfassend mindestens ein chirurgisches Bearbeitungswerkzeug (112), insbesondere einen Meißel (112), und mindestens ein chirurgisches Führungsinstrument (10) für das mindestens eine chirurgische Bearbeitungswerkzeug (112) mit einem Implantatkörper (24) zum Einführen in einen Zwischenwirbelraum (124) zwischen benachbarte Wirbelkörper (126) einer menschlichen oder tierischen Wirbelsäule (128), mit einem Schaft (18) und mit einer Anschlagvorrichtung (76) zum Begrenzen einer Einführtiefe des Führungsinstruments (10) in den Zwischenwirbelraum (124), wobei der Schaft (18) ein distales und ein proximales Ende (20, 52) aufweist, wobei der Implantatkörper (24) zwei an den benachbarten Wirbelkörpern (126) anlegbare Anlageflächen (26) definiert und am distalen Ende (52) des Schafts (18) angeordnet ist, wobei die Anschlagvorrichtung (76) mindestens einen am distalen Ende (52) des Schafts (18) oder am Implantatkörper (24) beweglich gelagerten Anschlag (76) aufweist, der eine quer oder im wesentlichen quer zu mindestens einer der Anlageflächen in distaler Richtung weisende Anschlagfläche (77) aufweist, **dadurch gekennzeichnet, dass** das mindestens eine chirurgische Führungsinstrument (10) ein Instrument (10) oder ein Satz nach einem der Ansprüche 1 bis 30 ist.

32. Instrumentarium nach Anspruch 31, **dadurch gekennzeichnet, dass** das mindestens eine Bearbeitungswerkzeug (112) ein Einfach- oder ein Doppelmeißel (112) ist.

33. Instrumentarium nach einem der Ansprüche 31 oder 32, **dadurch gekennzeichnet, dass** das mindestens einen Bearbeitungswerkzeug (112) einen hohlen Werkzeugschaft (116) aufweist, an dessen distalem Ende mindestens eine Werkzeugschneide (118) angeordnet ist und dass der Schaft (18) des Führungsinstruments (10) in den Werkzeugschaft (116) einführbar ist.
